# FASCICULE DE BREVET EUROPEEN

(11) **EP 3 917 604 B1**
(45) Date de publication et mention de la délivrance du brevet: **07.06.2023**
(21) Numéro de dépôt: 20701571.0
(22) Date de dépôt: 21.01.2020
(51) Int. Cl.: A61M 25/00, A61M 25/04, A61M 1/36, A61M 25/01, A61M 1/16

(54) **CANULE D'INJECTION, SYSTÈME ECMO**
INJEKTIONSKANÜLE, ECMO-SYSTEM
INJECTION CANNULA, ECMO SYSTEM

(30) Priorité: 28.01.2019 FR 1900752
(43) Date de publication de la demande: 08.12.2021
(73) Titulaire: ASSISTANCE PUBLIQUE, HOPITAUX DE PARIS, 75004 Paris (FR)
(72) Inventeur: MORDANT, Pierre, 75011 Paris (FR)
(74) Mandataire: Cabinet Laurent & Charras
(86) Numéro de dépôt international: PCT/EP2020/051350
(87) Numéro de publication internationale: WO 2020/156874

(56) Documents cités:
- WO-A2-2004/032791
- FR-A1- 3 058 642
- US-A- 5 542 936
- US-A1- 2016 121 079

## Description

### DOMAINE TECHNIQUE DE L'INVENTION

Le domaine technique de l'invention est celui des canules pour l'injection d'un fluide dans une cavité du corps humain ou animal, par exemple un organe, un vaisseau sanguin artériel ou veineux, un vaisseau lymphatique, ou encore une structure bronchique.

Plus particulièrement, l'invention se rapporte à une canule d'injection pour un système d'oxygénation par membrane extracorporelle, appelé système ECMO, ainsi qu'à un système ECMO comportant une telle canule d'injection.

### ARRIÈRE-PLAN TECHNOLOGIQUE DE L'INVENTION

Aujourd'hui, il existe des canules associées à des systèmes d'oxygénation par membrane extracorporelle, connus sous l'acronyme ECMO. En particulier, un système ECMO comprend un ensemble de composants permettant d'extraire, au moyen d'une canule d'admission, un volume sanguin d'une cavité du corps humain ou animal, de l'oxygéner, de le décarboxyler, éventuellement de le chauffer, puis de le réinjecter, au moyen d'une canule d'injection, dans une cavité différente ou identique à la cavité de prélèvement.

En particulier, il existe toutes sortes de canules de systèmes ECMO selon les applications souhaitées, notamment des applications veino-veineuse VV ou veino-artérielle VA ou encore veino-artério-veineuse VAV.

L'ECMO-VA, désignant « Extra Corporeal Membrane Oxygénation - Veno Arterial » pour ECMO veino-artérielle, est un traitement de référence du choc cardiogénique réfractaire, de l'arrêt cardiaque réfractaire, de la décompensation d'hypertension artérielle pulmonaire primitive ou secondaire. Il s'agit d'une technique privilégiée en cas de mauvaise tolérance hémodynamique ou respiratoire de la transplantation pulmonaire, de la transplantation cardiaque, et de toute chirurgie thoracique pulmonaire ou cardiovasculaire.

L'ECMO-VA est désignée comme une « ECMO-VA périphérique » lorsque l'injection de sang oxygéné est effectuée dans une artère périphérique et non dans la racine aortique ou dans l'artère pulmonaire (ECMO-VA centrale). Le circuit d'ECMO-VA périphérique comprend généralement une canule d'admission qui est préférentiellement placée dans une veine de grand diamètre, une pompe centrifuge, une membrane d'oxygénation et de décarboxylation et une canule d'injection qui est elle aussi placée préférentiellement dans une artère de grand diamètre, par exemple l'artère axillaire ou l'artère fémorale.

Cependant, l'inconvénient d'un tel système concerne la mise en place de la canule d'injection artérielle dans une artère périphérique du fait d'un risque important d'occlusion artérielle et donc d'ischémie de membre. En effet, une fois insérée dans l'artère, la canule d'injection peut entrainer une diminution du débit d'aval due à la présence de la canule et à la création d'un flux artériel rétrograde. Les termes « aval » et « rétrograde » sont définis par rapport au sens de circulation physiologique du sang. La diminution du débit d'aval peut alors engendrer une altération du flux artériel de perfusion du membre et créer une ischémie de membre.

Afin de pallier les inconvénients précités, il est connu de brancher une seconde canule dite de reperfusion en Y sur la ligne artérielle de l'ECMO. Cette canule de reperfusion est implantée dans le sens antérograde, en aval de la canule principale, généralement implantée dans le sens rétrograde. Cependant, cette technique comporte des inconvénients, notamment en raison de l'utilisation de deux canules de diamètres différents devant être implantées à deux endroits différents de l'artère ainsi que dans deux sens différents. Il s'agit donc d'une procédure longue et complexe, le plus souvent réalisée dans un contexte d'urgence.

Ainsi, une solution, décrite dans la demande de brevet FR1661037, consiste à utiliser une canule d'injection associant dans un seul dispositif une canule principale alors appelé lumière principale, et une canule de reperfusion alors appelée lumière accessoire, de manière à réduire le nombre de points d'insertion et donc de simplifier la procédure d'introduction de la canule d'injection dans l'artère. Afin que le fluide sortant de la lumière accessoire s'écoule dans un sens antérograde, la lumière accessoire de reperfusion de manière à capter une partie du flux sanguin rétrograde provenant de la lumière principale, puis présente une portion coudée permettant d'inverser la direction d'écoulement du fluide. Le fluide circulant dans la lumière accessoire est ensuite évacué dans l'artère dans le sens antérograde par un orifice de sortie ménagé dans la paroi de la canule d'injection.

La demande de brevet FR1661037 (Publication A1 FR3058642 du 18 Mai 2018) est considéré comme l'état de la technique le plus proche. US2016121079, US5542936 et WO2004032791 divulgue chacun une canule de l'état de la technique.

Afin d'assurer le bon fonctionnement d'une telle canule, il est indispensable que l'orifice de sortie de la lumière accessoire soit correctement positionné dans l'artère. En effet, s'il est mal positionné dans l'artère, l'orifice de sortie peut :
- se plaquer contre la paroi postérieure ou contre la paroi antérieure de l'artère ce qui ne permet pas d'assurer une reperfusion efficace et peut provoquer des lésions dans la paroi de l'artère dues à la force du flux sanguin injecté contre la paroi (dissection artérielle, thrombose, embols distaux),
- se retrouver à l'extérieur de l'artère et donc être inefficace, voire de provoquer un hématome sous-cutané majeur.

### RÉSUMÉ DE L'INVENTION

L'invention offre une solution aux problèmes évoqués précédemment, en permettant de positionner correctement l'orifice de sortie d'une lumière accessoire de reperfusion dans une cavité du corps humain ou animal.

Un premier aspect de l'invention concerne une canule pour l'injection d'un fluide dans une cavité du corps humain ou animal selon la revendication 1, comportant :
- une lumière principale définissant un premier espace de la canule pour la circulation du fluide selon une première direction,
- une lumière accessoire de reperfusion définissant un deuxième espace de la canule et comportant :
   o une entrée pour capter une partie du fluide s'écoulant selon la première direction,
   o une portion coudée pour modifier la direction d'écoulement du fluide capté par la lumière accessoire de reperfusion,
   o une sortie pour évacuer le fluide capté selon une deuxième direction dans la cavité.

En outre, la canule comporte un dispositif de positionnement de la canule dans la cavité, le dispositif de positionnement comportant une butée et des moyens de déplacement configurés pour déplacer ladite butée le long d'une lumière auxiliaire, la lumière auxiliaire définissant un troisième espace de la canule et comportant une sortie débouchant dans la cavité. Les moyens de déplacement permettent de déployer la butée dans la cavité, via la sortie de la lumière auxiliaire, pour assurer le blocage en position de la canule dans la cavité.

De plus, la sortie de la lumière auxiliaire est agencée à une distance prédéterminée de la sortie de la lumière accessoire de reperfusion de sorte que lorsque la butée bloque en position la canule dans la cavité, la sortie de la lumière accessoire de reperfusion soit orientée dans la cavité de manière que le fluide capté soit évacué selon la deuxième direction.

Par « cavité du corps », on entend un organe, un vaisseau sanguin, un vaisseau lymphatique ou encore une structure bronchique.

Grâce à la canule d'injection selon l'invention, la sortie de la lumière accessoire de reperfusion est correctement positionnée dans la cavité ce qui permet d'assurer une reperfusion efficace de la cavité sans nécessité de contrôle radiologique ou échographique.

En outre, la distance entre la sortie de la lumière accessoire de reperfusion et la sortie de la lumière auxiliaire est choisie selon les dimensions de la cavité de sorte que, lorsque la butée bloque la position de la canule d'injection dans la cavité, la sortie de la lumière accessoire de reperfusion soit correctement positionnée dans la cavité. Par « correctement positionnée », on entend que la sortie de la lumière accessoire de reperfusion est agencée dans la cavité de sorte que le flux sanguin soit évacué en pleine cavité selon la deuxième direction. En particulier, la distance prédéterminée est choisie de sorte que la sortie de la lumière accessoire de reperfusion ne puisse pas se plaquer contre la paroi de la cavité ce qui empêcherait une reperfusion efficace de la cavité. De plus, un tel positionnement de la sortie de la lumière accessoire de reperfusion dans la cavité permet d'éviter de provoquer des lésions dans la paroi de la cavité dues à la force du flux sanguin injecté contre la paroi.

Par ailleurs, la butée déployée permet de bloquer en position la canule d'injection dans la cavité de sorte qu'elle ne risque pas de s'extraire du corps du patient même en cas de mouvements du patient. Ainsi, la canule d'injection peut être maintenue dans le corps d'un patient pendant une longue durée.

En outre, le blocage de la canule d'injection dans la cavité permet de s'assurer que la sortie de la lumière accessoire de reperfusion ne puisse pas se retrouver à l'extérieur de la cavité et donc d'être inefficace ou encore de provoquer un hématome sous-cutané majeur.

Outre les caractéristiques qui viennent d'être évoquées dans le paragraphe précédent, la canule d'injection selon le premier aspect de l'invention peut présenter une ou plusieurs caractéristiques complémentaires parmi les suivantes, considérées individuellement ou selon toutes les combinaisons techniquement possibles :
- la distance prédéterminée entre la sortie de la lumière accessoire de reperfusion et la sortie de la lumière auxiliaire est comprise entre 0,1 et 500 mm ;
- les moyens de déplacement comportent un bouton-poussoir apte à se déplacer dans une rainure longitudinale ménagée dans la canule, le déplacement du bouton-poussoir dans la rainure longitudinale entrainant le déplacement de la butée le long de la lumière auxiliaire via un élément de liaison relié à ladite butée ;
- la butée déployée présente une forme et des dimensions adaptées pour empêcher le retrait de la canule lorsque ladite canule est à l'intérieur de la cavité ;
- la butée est réalisée dans un matériau à mémoire de forme ;
- la butée est réalisée à partir d'un fil en alliage nickel-titane ;
- la butée déployée présente la forme d'un losange ;
- la butée présente un revêtement anti-thrombogène et/ou anti-proliférant ;
- la butée et/ou la sortie de la lumière auxiliaire présente(nt) un marquage radio-opaque ou écho-opaque adapté pour assurer une identification de ladite butée et/ou de ladite sortie de la lumière auxiliaire par radiographie ou échographie ;
- un robinet de purge est raccordé à la lumière accessoire de reperfusion.

Un second aspect de l'invention concerne un système d'injection d'un fluide dans une cavité du corps humain ou animal selon la revendication 11, comportant :
- une canule d'admission pour recevoir un fluide prédéterminé ;
- une pompe pour assurer le pompage du fluide prédéterminé provenant de la canule d'admission ;
- un oxygénateur pour assurer l'oxygénation du fluide prédéterminé provenant d'une sortie de la pompe ;
- une canule d'injection selon le premier aspect de l'invention, pour injecter le fluide oxygéné dans la cavité.

Selon un mode de réalisation non limitatif, le système d'injection comporte un échangeur de chaleur pour injecter le fluide oxygéné à une température prédéterminée dans la canule d'injection.

L'invention et ses différentes applications seront mieux comprises à la lecture de la description qui suit et à l'examen des figures qui l'accompagnent.

### BRÈVE DESCRIPTION DES FIGURES

Les figures sont présentées à titre indicatif et nullement limitatif de l'invention.
- La figure 1 montre une représentation schématique d'un système ECMO selon un mode de réalisation de l'invention ;
- La figure 2 représente une canule d'injection selon un mode de réalisation de l'invention, utilisée dans le système ECMO illustré à la figure 1 ;
- La figure 3 est un agrandissement de la canule d'injection illustrée à la figure 2, au niveau des moyens de déplacement de la butée ;
- La figure 4 est un agrandissement de la canule d'injection illustrée à la figure 2, au niveau de la butée ;
- La figure 5 est un schéma illustrant l'introduction de la canule d'injection de la figure 2 dans une artère ;
- La figure 6 montre un agrandissement de la canule d'injection de la figure 5, au niveau du point d'insertion de la canule dans l'artère.

Les figures sont présentées à titre indicatif et nullement limitatif de l'invention.

### DESCRIPTION DETAILLÉE

L'invention se rapporte à une canule d'injection utilisée pour injecter un fluide dans une cavité du corps humain ou animal, par exemple un organe, un vaisseau sanguin, un vaisseau lymphatique ou encore une structure bronchique. En particulier, la canule d'injection est utilisée dans un système ECMO qui permet d'oxygéner un volume de fluide prélevé dans une cavité du corps et de le réinjecter dans une cavité différente ou identique à la cavité de prélèvement.

Dans la suite de la description, on admettra que le fluide à injecter dans une cavité du corps est du sang. En outre, on considère que le système ECMO est veino-artériel (VA) c'est-à-dire que la cavité du corps dans laquelle le flux est prélevé est une veine tandis que la cavité dans laquelle le flux est réinjecté est une artère. Naturellement, ce mode de réalisation n'est nullement limitatif, l'ECMO pouvant être par exemple de type veino-veineux (VV) ou encore veino-artério-veineux (VAV).

De plus, on nomme « direction d'écoulement antérograde » d'un fluide la direction d'écoulement physiologique S du sang dans un vaisseau, un organe. En outre, on nomme une « direction d'écoulement rétrograde » d'un fluide la direction inverse d'écoulement physiologique S du sang dans un vaisseau ou un organe. Le référentiel est donc pris par rapport à la physiologie du corps humain ou animal. Par extension de langage et pour la clarté de l'exposé du mode de réalisation, on nommera la direction d'écoulement de la lumière principale LP : la « direction rétrograde » et la direction d'écoulement de la lumière accessoire de reperfusion LA : la « direction antérograde », chacune étant considérée en sortie de la canule d'injection lorsque l'on évoque leur direction.

Par ailleurs, les termes « amont » et « aval » sont définis dans le présent texte par rapport au praticien qui manipule la canule d'injection selon l'invention.

Enfin, on désigne par « F0 », un flux de sang non traité c'est à dire pauvre en oxygène et riche en dioxyde de carbone et par « F1 », un flux de sang traité c'est-à-dire oxygéné et décarboxylé.

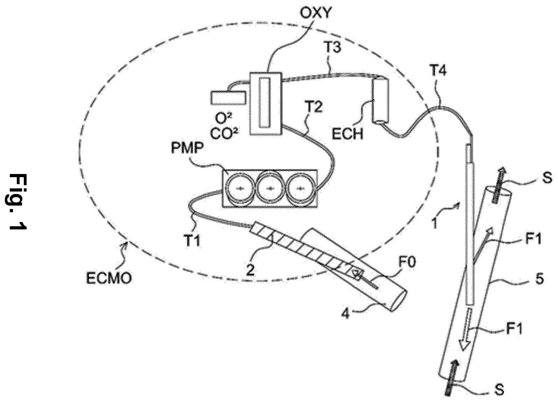

La figure 1 illustre un système ECMO comportant une canule d'injection selon l'invention.

En référence à la figure 1, le système ECMO comporte différents composants notamment une canule d'admission 2, une pompe PMP, un oxygénateur OXY, une canule d'injection 1, et un échangeur de chaleur ECH. Dans une variante de réalisation non illustrée, le système ECMO ne comporte pas d'échangeur de chaleur.

La pompe PMP assure une première fonction de pompage d'un volume de sang F0 pauvre en oxygène et riche en dioxyde de carbone de la canule d'admission 2 insérée dans une veine 4 du patient. Le volume de sang F0 est pompé à un débit prédéfini et possiblement paramétrable. En particulier, le débit doit permettre une oxygénation et une décarboxylation efficace du sang pauvre en oxygène et riche en dioxyde de carbone.

En outre, la pompe PMP assure une seconde fonction d'injection d'un volume de sang F1 oxygéné et décarboxylé dans la canule d'injection 1 positionnée dans une artère 5 du patient. De manière avantageuse, la pompe PMP est configurée pour que le sang injecté dans la canule d'injection 1 arrive sensiblement à la valeur d'un débit physiologique selon l'état du patient.

Par ailleurs, la pompe PMP peut être de type « centrifuge » c'est-à-dire qu'elle utilise le mouvement de rotation d'une roue insérée dans la pompe PMP. Dans une variante de réalisation, la pompe PMP est « à galets », appelée également « péristaltique ». En outre, la pompe PMP peut générer un débit pulsé.

Selon un mode de réalisation non illustré, le système ECMO comprend un réservoir qui permet de réguler le débit de sang traité. Le flux de sang F0 prélevé par la canule d'admission 2 est alors acheminé jusqu'au réservoir. De préférence, cette étape est réalisée avant l'étape de pompage.

L'oxygénateur OXY est relié à la pompe PMP à partir de laquelle il reçoit le sang F0 pauvre en oxygène et riche en dioxyde de carbone à un débit fixé. À cet effet, l'oxygénateur OXY comporte une membrane qui reproduit artificiellement la fonction de la membrane alvéolo-capillaire en réalisant des échanges gazeux permettant d'oxygéner le sang et d'éliminer le dioxyde de carbone contenu dans le sang.

Selon un mode de réalisation, la membrane de l'oxygénateur OXY est plane. Les oxygénateurs OXY à membranes planes comportent des membranes en silicone ou des membranes assemblées en couches. Selon un autre mode de réalisation, la membrane de l'oxygénateur OXY est tubulaire. Les membranes tubulaires sont composées de fibres creuses composées, par exemple de polyméthylpentènes non poreuses. Avantageusement, les fibres peuvent comprendre un revêtement offrant moins de résistance à l'écoulement et favorisant un écoulement de flux laminaire.

Par ailleurs, l'échangeur de chaleur ECH reçoit le volume de sang F1 oxygéné et décarboxylé de l'oxygénateur OXY pour le réchauffer avant d'atteindre la canule d'injection 1. En particulier, le volume de sang F1 passe par un système permettant de transférer l'énergie thermique d'un fluide tel que l'eau vers le sang, sans mélanger les deux fluides. Le flux thermique traverse la surface d'échange qui sépare le sang oxygéné et l'eau. L'échangeur de chaleur ECH fixe la température du sang afin qu'elle soit comprise dans l'intervalle de température du sang circulant dans le corps du patient.

L'échangeur de chaleur ECH peut être intégré au système ECMO ou être externe à ce dernier. En outre, selon un mode de réalisation, l'échangeur de chaleur ECH est relié à un réservoir de sorte à réchauffer le sang après son drainage, les étapes de pompage puis d'oxygénation/décarboxylation sont alors réalisées par la suite.

De manière alternative, le réchauffage du volume de sang F1 peut être réalisé par l'oxygénateur OXY lui-même. Dans ce cas, l'oxygénateur OXY comprend avantageusement des résistances prévues à cet effet.

La canule d'admission 2, la pompe PMP, l'oxygénateur OXY, l'échangeur de chaleur ECH et la canule d'injection 1 sont reliés entre eux par l'intermédiaire de tubes T1, T2, T3 et T4. Ces tubes T1, T2, T3, T4 assurent la circulation du sang oxygéné ou non, décarboxylé ou non, réchauffé ou non, entre les différents composants permettant son traitement. Ainsi, le premier tube T1 permet de relier la canule d'admission 2 à la pompe PMP. Le deuxième tube T2 permet de relier la pompe PMP à l'oxygénateur OXY. Le troisième tube T3 permet de relier l'oxygénateur OXY à l'échangeur de chaleur ECH et le quatrième tube T4 permet de relier l'échangeur de chaleur ECH à la canule d'injection 1.

Avantageusement, le diamètre des tubes T1, T2, T3, T4 est choisi de manière à permettre au sang de se déplacer tout en minimisant les risques d'hémolyse et de coagulation. En d'autres termes, le diamètre des tubes T1, T2, T3, T4 est ajusté selon les débits connus permettant la circulation du sang dans le corps.

En outre, les tubes T1, T2, T3, T4 présentent, de préférence, un revêtement anticoagulant, par exemple en héparine, afin d'éviter la formation de caillots de sang à l'intérieur des tubes T1, T2, T3, T4. De plus, les tubes T1, T2, T3, T4 peuvent également présenter un revêtement antiagrégant plaquettaire afin d'éviter l'agrégation plaquettaire et d'inhiber la formation d'un thrombus.

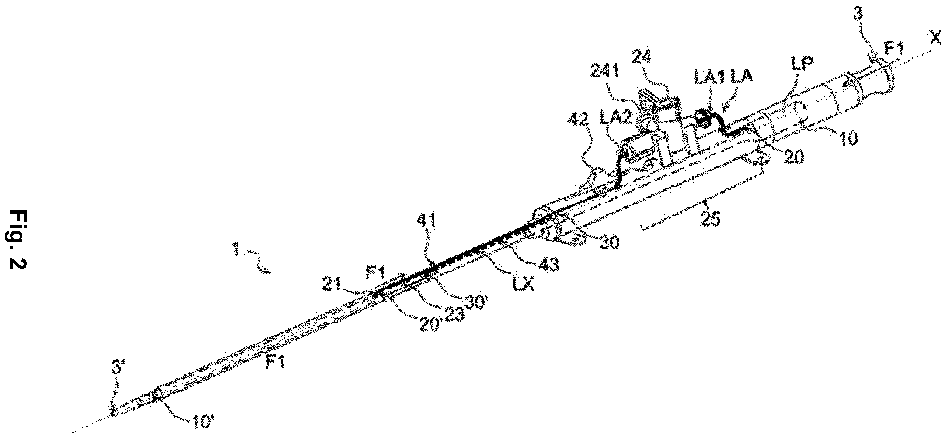

La figure 2 représente une vue en perspective de la canule d'injection 1 selon un mode de réalisation de l'invention, avant son insertion dans l'artère 5.

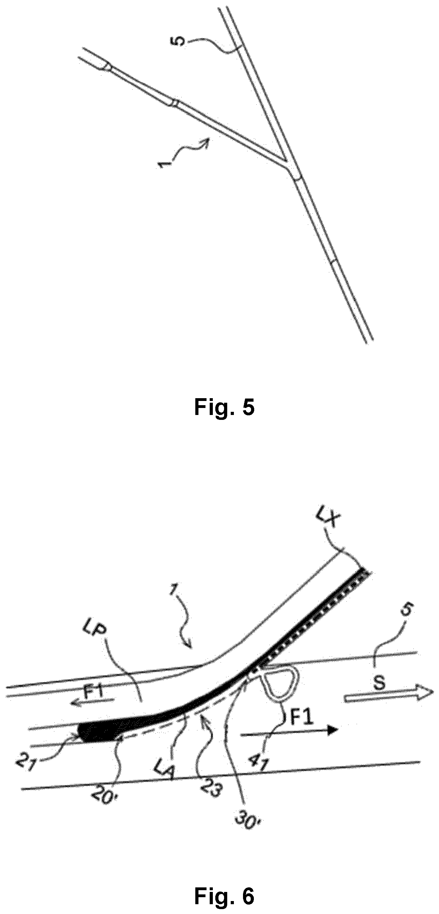

La figure 5 est un schéma illustrant l'introduction de la canule d'injection 1 de la figure 2 dans une artère 5.

La figure 6 est un agrandissement de la canule d'injection 1 illustrée à la figure 5, au niveau du point d'insertion de la canule 1 dans l'artère 5.

Lors de son introduction dans l'artère 5, la canule d'injection 1 est déformée de manière à pouvoir l'insérer transversalement dans la paroi de l'artère 5 et ainsi éviter d'engendrer des lésions dans la paroi de l'artère 5. Avantageusement, la canule d'injection 1 est réalisée à partir d'un matériau déformable, par exemple en polyuréthane. En outre, comme on peut le voir sur les figures 5 et 6, la canule d'injection 1 n'est que partiellement introduite dans l'artère 5, une partie de la canule d'injection 1 étant maintenue à l'extérieur du corps du patient pour pouvoir être manipulée par un praticien.

En référence à la figure 2, la canule d'injection 1, avant son insertion dans une artère 5, s'étend longitudinalement selon un axe X. En outre, la canule d'injection 1 comporte une entrée 3 apte à coopérer avec une embouchure de liaison (non représentée) provenant d'un composant de l'ECMO afin de recevoir le flux sanguin F1 traité. En outre, la canule d'injection 1 comporte une sortie 3' destinée à être insérée dans l'artère 5.

En outre, la canule d'injection 1 comporte une lumière principale LP, une lumière accessoire de reperfusion LA ainsi qu'une lumière auxiliaire LX délimitant trois espaces de la canule d'injection 1.

La lumière principale LP assure l'injection du flux sanguin F1 provenant de l'entrée 3 de la canule d'injection 1 dans l'artère 5, selon une première direction, ici une direction rétrograde. À cet effet, la lumière principale LP s'étend le long de la canule d'injection 1 et comporte une entrée 10 et une sortie 10'. L'entrée 10 de la lumière principale LP est agencée en aval de l'entrée 3 de la canule d'injection 1 afin de recevoir le flux sanguin F1. Le flux sanguin F1 est ensuite évacué par la sortie 10' de la lumière principale LP agencée en amont de la sortie 3' de la canule d'injection 1.

Par ailleurs, la lumière principale LP est de section circulaire et présente un diamètre qui varie entre l'entrée 10 et la sortie 10' de ladite lumière principale LP. En outre, le diamètre de la lumière principale LP au niveau de sa sortie 10' est plus faible qu'au niveau de son entrée 10 afin que la partie de la canule d'injection 1 destinée à être insérée dans l'artère 5 soit adaptée au diamètre de ladite artère 5. De plus, une telle réduction du diamètre de la lumière principale LP permet d'accélérer les débits injectés et donc de diminuer l'effort hydrodynamique à fournir par le système ECMO. Avantageusement, le diamètre de la lumière principale LP est compris entre 10F et 21F, i.e. entre 3.3 et 7mm. Naturellement, le diamètre de lumière principale LP peut être constant le long de la lumière principale LP. En outre, la lumière principale LP pourrait tout à fait présenter une section autre que circulaire, par exemple une section ovale, elliptique.

La lumière accessoire de reperfusion LA assure l'injection d'une partie du flux sanguin F1 dans l'artère 5, selon une deuxième direction, ici une direction antérograde. À cet effet, la lumière accessoire de reperfusion LA présente une entrée 20 en communication fluidique avec la lumière principale LP. En particulier, l'entrée 20 de la lumière accessoire de reperfusion LA est agencée en aval de l'entrée 10 de la lumière principale LP afin de capter une partie du flux sanguin F1 provenant de la lumière principale LP. Dans une variante de réalisation, l'entrée 20 de la lumière accessoire de reperfusion LA n'est pas en communication fluidique avec la lumière principale LP. Dans ce cas, la lumière accessoire de reperfusion LA capte une partie du flux sanguin F1 directement depuis l'entrée 3 de la canule d'injection 1. En outre, comme on peut le voir sur la figure 2, une portion de la lumière accessoire de reperfusion LA s'étend parallèlement à la lumière principale LP.

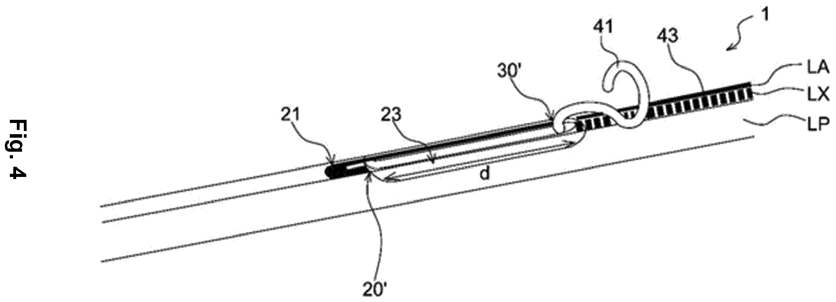

Par ailleurs, la lumière accessoire de reperfusion LA comporte une portion coudée 21 permettant de modifier la direction d'écoulement rétrograde du flux sanguin F1 capté par la lumière accessoire de reperfusion LA. La portion coudée 21 permet au flux sanguin F1 capté de s'écouler selon une direction antérograde. Comme on peut le voir sur la figure 4, la portion coudée 21 forme un arc de cercle, voire un demi-cercle permettant de modifier l'orientation du fluide F1 dans une direction antérograde, c'est-à-dire dans une direction opposée à la direction d'éjection du flux sanguin F1 en sortie 10' de la lumière principale LP.

La lumière accessoire de reperfusion LA comporte, en outre, une sortie 20' débouchant dans une ouverture latérale 23 ménagée dans la canule d'injection 1 de manière à évacuer le flux sanguin F1 s'écoulant selon une direction antérograde dans l'artère 5.

La lumière accessoire de reperfusion LA, également de section circulaire, présente, à la différence de la lumière principale LP, un diamètre constant sur toute sa longueur. Selon un exemple de réalisation, le diamètre de la lumière accessoire de reperfusion LA présente un diamètre compris entre 20G, i.e. 0.8mm, et 6F, i.e. 2mm. Naturellement, le diamètre de la lumière accessoire de reperfusion LA pourrait varier entre l'entrée 20 et la sortie 20' de la lumière accessoire de reperfusion LA. On note qu'il est possible de faire varier le rapport des sections des lumières LA et LP afin de contrôler la vitesse et le débit d'éjection du flux sanguin F1 dans l'artère 5 dans les deux directions, i.e. antérograde et rétrograde. En outre, la lumière accessoire de reperfusion LA pourrait présenter une section autre que circulaire, par exemple ovale, elliptique.

Dans une variante de réalisation non illustrée, la lumière accessoire de reperfusion LA est intégrée à l'intérieur de la lumière principale LP tel que décrit dans la demande de brevet FR1661037. Dans ce cas, la sortie 20' de la lumière accessoire de reperfusion LA débouche également dans l'ouverture latérale 23 ménagée dans la canule d'injection 1. Dans une variante de réalisation non illustrée, la sortie 20' de la lumière accessoire de reperfusion LA est équipée d'un dispositif électromécanique de mesure du flux connecté à un affichage électronique situé au niveau de la poignée, i.e. au niveau de l'entrée 3, de la canule d'injection 1.

Par ailleurs, un robinet de purge 24 est raccordé à la lumière accessoire de reperfusion LA afin d'assurer sa purge. En particulier, le robinet de purge 24 est un robinet à 3 voies dans lequel deux voies sont raccordées à deux portions LA1, LA2 de la lumière accessoire de reperfusion LA tandis qu'une voie forme une sortie 241. On note que la partie de la canule d'injection 1 comportant le robinet de purge 24 est destinée à être à maintenue à l'extérieur du corps du patient.

Le robinet de purge 24 permet l'ouverture de deux voies et la fermeture de la troisième voie, de manière simultanée. Ainsi, lorsque la sortie 241 du robinet de purge 24 est fermée, le flux sanguin F1 circulant dans la lumière accessoire de reperfusion LA peut passer de l'entrée 20 de la lumière accessoire de reperfusion LA à la sortie 20' de la lumière accessoire de reperfusion LA. En outre, lorsque la sortie 241 du robinet de purge 24 est ouverte, le flux sanguin F1 ne peut pas passer de l'entrée 20 de la lumière accessoire de reperfusion LA à la sortie 20' de la lumière accessoire de reperfusion LA.

Dans une première position du robinet de purge 24, dite de purge antérograde ou de contrôle du flux, le flux sanguin F1 circule dans la lumière accessoire de reperfusion LA de l'amont vers l'aval, i.e. de l'entrée 20 de la lumière accessoire de reperfusion LA vers la sortie 241 du robinet de purge 24.

Dans une seconde position du robinet de purge 24, dite de purge rétrograde ou de contrôle du reflux, le flux sanguin F1 circule dans la lumière accessoire de reperfusion LA de l'aval vers l'amont, i.e. de la sortie 20' de la lumière accessoire de reperfusion LA vers la sortie 241 du robinet de purge 24. On note que le flux sanguin F1 circulant dans la lumière accessoire de reperfusion LA de l'aval vers l'amont peut être généré lors de l'introduction de la canule d'injection 1 dans l'artère 5.

Avantageusement, le robinet de purge 24 permet de s'assurer que la sortie 20' de la lumière accessoire de reperfusion LA est correctement positionnée dans l'artère 5. Pour ce faire, le flux et le reflux entre la sortie 20' de la lumière accessoire de reperfusion LA et la sortie 241 du robinet de purge 24 sont testés en utilisant une seringue branchée à la sortie 241 du robinet de purge 24 afin, respectivement, d'injecter ou d'aspirer un fluide entre la sortie 20' de la lumière accessoire LA et la sortie 241 du robinet de purge 24.

Par ailleurs, afin d'assurer une reperfusion efficace de l'artère 5, il est primordial que la sortie 20' de la lumière accessoire de reperfusion LA soit positionnée dans l'artère 5 de sorte que le flux sanguin F1 s'écoulant selon une direction antérograde soit éjecté en pleine lumière de l'artère 5. En d'autres termes, la sortie 20' de la lumière accessoire de reperfusion LA ne doit pas être positionnée de sorte à faire face à une paroi antérieure, postérieure ou latérale de l'artère 5.

Pour ce faire, la canule d'injection 1 comporte un dispositif de positionnement de la canule d'injection 1 dans l'artère 5 qui permet de bloquer en position la canule d'injection 1 dans l'artère 5. De plus, la sortie 30' de la lumière auxiliaire LX est positionnée à une distance prédéterminée d de la sortie 20' de la lumière accessoire de reperfusion LA de sorte que, lorsque la canule d'injection 1 est bloquée en position dans l'artère 5, le flux sanguin F1 qui circule dans la lumière accessoire de reperfusion LA soit évacué correctement dans l'artère 5, i.e. selon la direction antérograde. Avantageusement, la distance prédéterminée d entre la sortie 30' de la lumière auxiliaire LX et la sortie 20' de la lumière accessoire de reperfusion LA est comprise entre 0,1 et 500mm.

Afin de bloquer la position de la canule d'injection 1 dans l'artère 5, le dispositif de positionnement comporte une butée 41 adaptée pour se déplacer le long d'une lumière auxiliaire LX et à se déployer dans l'artère 5.

La lumière auxiliaire LX s'étend parallèlement à la lumière principale LP et présente une entrée 30 et une sortie 30'. La sortie 30' de la lumière auxiliaire LX, agencée en amont de la sortie 20' de la lumière accessoire de reperfusion LA, débouche dans l'ouverture latérale 23 ménagée dans la canule d'injection 1. Dans une variante de réalisation, la sortie 30' de la lumière auxiliaire LX et la sortie 20' de la lumière accessoire de reperfusion LA ne débouchent pas dans la même ouverture latérale de la canule d'injection 1.

En outre, la lumière auxiliaire LX est de section circulaire et présente un diamètre constant sur toute sa longueur, par exemple un diamètre compris entre 20G et 6F, i.e. entre 0.8 et 2mm. Dans une variante de réalisation, le diamètre de la lumière auxiliaire LX varie entre l'entrée 30 et la sortie 30' de la lumière auxiliaire LX. On note, en outre, que la lumière auxiliaire LX peut présenter une section autre que circulaire, par exemple elliptique.

Le déplacement de la butée 41 le long de la lumière auxiliaire LX puis son déploiement dans l'artère 5 sont contrôlés à distance par des moyens de déplacement destinés à être maintenus à l'extérieur du corps du patient. En particulier, les moyens de déplacement assurent le déplacement de la butée 41 via un élément de liaison 43, relié à la butée 41, apte à se déplacer le long de la lumière auxiliaire LX.

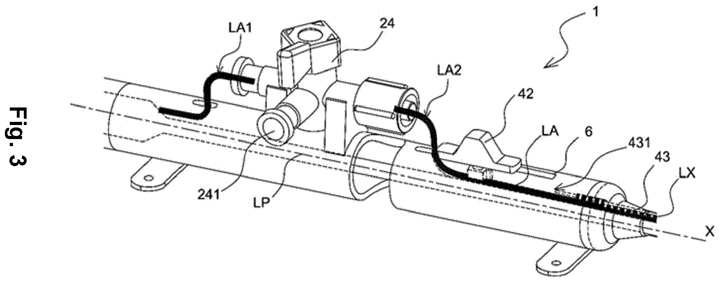

La figure 3 est un agrandissement de la canule d'injection 1 au niveau des moyens de déplacement de la butée 41.

Selon le mode de réalisation illustré aux figures 2 et 3, les moyens de déplacement sont formés par un bouton-poussoir 42 adapté pour coulisser le long d'une rainure longitudinale 6 ménagée dans la lumière principale LP. Le déplacement, d'amont en aval, du bouton-poussoir 42 dans la rainure longitudinale 62 entraine le déplacement des moyens de liaison 43 et donc de la butée 41 dans le même sens. En particulier, lors du déplacement du bouton-poussoir 42 de l'amont vers l'aval, ce dernier vient au contact d'une extrémité 431 des moyens de liaison 43 puis pousse les moyens de liaison 43 et la butée 41 vers l'artère 5. À la sortie 30' de la lumière auxiliaire LX, la butée 41 se déploie dans l'artère 5. Dans une variante de réalisation, le déploiement de la butée 41 est réalisé au moyen d'un moteur électrique situé au niveau de la poignée de la canule d'injection 1, sous le contrôle d'un interrupteur également situé au niveau de ladite poignée.

La figure 4 est un agrandissement de la canule d'injection 1, au niveau de la butée 41.

La forme et les dimensions de la butée 41 sont choisies de sorte qu'une fois la butée 41 déployée dans l'artère 5, la canule d'injection 1 ne puisse pas être retirée de l'artère 5. Comme on peut le voir sur les figures 4 et 6, la butée 41 déployée s'étend en direction de l'amont de la canule d'injection 1 en formant un coude avec les moyens de liaison 43 permettant de bloquer la position de la canule d'injection 1 dans l'artère 5. En particulier, après le déploiement de la butée 41 dans l'artère 5, la canule d'injection 1 doit être retirée de l'artère 5 jusqu'à ce que la butée 41 vienne se plaquer contre la face interne de la paroi antérieure de l'artère 5. Dans cette position, la butée 41 empêche le retrait de la canule d'injection 1 de l'artère 5. Les termes « antérieur » et « postérieur », sont utilisés pour désigner, respectivement, une partie avant et une partie arrière d'une cavité ou d'un organe du corps humain en position anatomique.

Avantageusement, la butée 41 est réalisée dans un matériau à mémoire de forme c'est-à-dire que le matériau a la capacité de garder en mémoire une forme initiale et de reprendre sa forme initiale même après une déformation. Ainsi, la butée 41 est adaptée pour se déformer lorsqu'elle traverse la lumière auxiliaire LX et pour reprendre sa forme initiale lorsqu'elle est à l'extérieur de la lumière auxiliaire LX, notamment à l'intérieur de l'artère 5.

En outre, la forme de la butée 41 est choisie de manière à ne pas engendrer de lésions dans l'artère 5 tout en présentant une résistance à la traction. Avantageusement, la butée 41 présente des angles arrondis ainsi qu'une forme ramassée. Par ailleurs, la forme de la butée 41 peut varier afin d'obtenir la meilleure résistance avec le plus faible encombrement et la moindre agressivité pour la paroi de l'artère 5.

De préférence, la butée 41 et/ou les moyens de liaison 43 sont réalisés à partir d'un fil en alliage nickel-titane car il présente une bonne rigidité. En outre, la butée 41 peut présenter un revêtement anti-thrombogène, i.e. anti-coagulant et/ou antiagrégants plaquettaires et/ou un revêtement anti-proliférant.

Par ailleurs, afin de retirer la canule d'injection 1 de l'artère 5, il suffit de déplacer le bouton poussoir 42, de l'aval vers l'amont, ce qui entraine le retrait de la butée 41 qui se déforme avant de se réintroduire dans la lumière auxiliaire LX.

Avantageusement, la butée 41 présente un marquage radio-opaque ou écho-opaque adapté pour assurer une identification de la butée 41 par radiographie ou échographie. Selon un autre mode de réalisation, le marquage d'identification est réalisé sur la sortie 30' de la lumière auxiliaire LX. Dans une autre variante de réalisation, la butée 41 et la sortie 30' de la lumière auxiliaire LX sont toutes les deux marquées.

L'invention se rapporte également à un système ECMO comportant la canule d'injection 1 selon l'invention.

## Revendications

1. Canule (1) pour l'injection d'un fluide (F1) dans une cavité (5) du corps humain ou animal, comportant :
- une lumière principale (LP) définissant un premier espace de la canule (1) pour la circulation du fluide (F1) selon une première direction,
- une lumière accessoire de reperfusion (LA) définissant un deuxième espace de la canule (1) et comportant:
∘ une entrée (20) pour capter une partie du fluide (F1) s'écoulant selon la première direction,
o une portion coudée (21) pour modifier la direction d'écoulement du fluide (F1) capté par la lumière accessoire de reperfusion (LA),
o une sortie (20') pour évacuer le fluide (F1) capté selon une deuxième direction dans la cavité (5),
**caractérisée en ce que** la canule (1) comporte un dispositif de positionnement de la canule (1) dans la cavité (5), le dispositif de positionnement comportant une butée (41) et des moyens de déplacement configurés pour déplacer ladite butée (41) le long d'une lumière auxiliaire (LX), la lumière auxiliaire (LX) définissant un troisième espace de la canule (1) et comportant une sortie (30') débouchant dans la cavité (5),
les moyens de déplacement permettant de déployer la butée (41) dans la cavité (5), via la sortie (30') de la lumière auxiliaire (LX), pour assurer le blocage en position de la canule (1) dans la cavité (5),
la sortie (30') de la lumière auxiliaire (LX) étant agencée à une distance prédéterminée (d) de la sortie (20') de la lumière accessoire de reperfusion (LA) de sorte que lorsque la butée (41) bloque en position la canule (1) dans la cavité (5), la sortie (20') de la lumière accessoire de reperfusion (LA) soit orientée dans la cavité (5) de sorte que le fluide (F1) capté soit évacué selon la deuxième direction.

2. Canule (1) d'injection selon la revendication précédente, **caractérisée en ce que** la distance prédéterminée (d) entre la sortie (30') de la lumière auxiliaire (LX) et la sortie (20') de la lumière accessoire de reperfusion (LA) est comprise entre 0,1 et 500mm.

3. Canule (1) d'injection selon l'une quelconque des revendications précédentes, **caractérisée en ce que** les moyens de déplacement comportent un bouton-poussoir (42) apte à se déplacer dans une rainure longitudinale (6) ménagée dans la canule (1), le déplacement du bouton-poussoir (42) dans la rainure longitudinale (6) entrainant le déplacement de la butée (41) le long de la lumière auxiliaire (LX) via un élément de liaison (43) relié à ladite butée (41).

4. Canule (1) d'injection selon l'une des revendications précédentes, **caractérisée en ce que** la butée (41) déployée présente une forme et des dimensions adaptées pour empêcher le retrait de la canule (1) lorsqu'elle est insérée à l'intérieur de la cavité (5).

5. Canule (1) d'injection selon l'une quelconque des revendications précédentes, **caractérisée en ce que** la butée (41) est réalisée dans un matériau à mémoire de forme.

6. Canule (1) d'injection selon l'une quelconque des revendications précédentes, **caractérisée en ce que** la butée (41) est réalisée à partir d'un fil en alliage nickel-titane.

7. Canule (1) d'injection selon l'une quelconque des revendications précédentes, **caractérisée en ce que** la butée (41) déployée présente la forme d'un losange.

8. Canule (1) d'injection selon l'une quelconque des revendications précédentes, **caractérisée en ce que** la butée (41) présente un revêtement anti-thrombogène et/ou anti-proliférante.

9. Canule (1) d'injection selon l'une quelconque des revendications précédentes, **caractérisée en ce que** la butée (41) et/ou la sortie (30') de la lumière auxiliaire (LX) présente(nt) un marquage radio-opaque ou écho-opaque adapté pour assurer une identification de ladite butée (41) et/ou la sortie (30') de ladite lumière auxiliaire (LX) par radiographie ou échographie.

10. Canule (1) d'injection selon l'une quelconque des revendications précédentes, **caractérisée en ce qu'**elle comporte un robinet de purge (24) raccordé à la lumière accessoire de reperfusion (LA).

11. Système d'injection d'un fluide (F1) dans une cavité (5) du corps humain ou animal, **caractérisé en ce qu'**il comporte :
- une canule d'admission (2) pour recevoir un fluide prédéterminé (F0) ;
- une pompe (PMP) pour assurer le pompage du fluide prédéterminé (F0) provenant de la canule d'admission (2) ;
- un oxygénateur (OXY) pour assurer l'oxygénation du fluide prédéterminé (F0) provenant d'une sortie de la pompe (PMP) ;
- une canule d'injection (1) selon l'une quelconque des revendications précédentes pour injecter le fluide (F1) oxygéné dans la cavité (5).

## Patentansprüche

1. Kanüle (1) zur Injektion einer Flüssigkeit (F1) in einen Hohlraum (5) des menschlichen oder tierischen Körpers, die enthält:
- eine Hauptöffnung (LP), die einen ersten Raum der Kanüle (1) definiert, zur Zirkulation der Flüssigkeit (F1) in einer ersten Richtung,
- eine zusätzliche Reperfusionsöffnung (LA), die einen zweiten Raum der Kanüle (1) definiert und enthält:
o einen Eingang (20) zum Auffangen eines Teils der Flüssigkeit (F 1), die in die erste Richtung fließt;
∘ einen abgewinkelten Abschnitt (21) zur Änderung der Fließrichtung der Flüssigkeit (F1), die von der zusätzlichen Reperfusionsöffnung (LA) aufgefangen wird,
∘ einen Ausgang (20') zur Entfernung der Flüssigkeit (F1), die in einer zweiten Richtung im Hohlraum (5) aufgefangen wird,
**dadurch gekennzeichnet, dass** die Kanüle (1) eine Positionierungsvorrichtung der Kanüle (1) im Hohlraum (5) enthält, dabei enthält die Positionierungsvorrichtung einen Anschlag (41) und Verschiebevorrichtungen, die dazu konfiguriert sind, diesen Anschlag (41) entlang einer Hilfsöffnung (LX) zu verschieben, dabei definiert die Hilfsöffnung (LX) einen dritten Raum der Kanüle (1) und enthält einen Ausgang (30'), der im Hohlraum (5) mündet,
mit den Verschiebevorrichtungen ist es möglich, den Anschlag (41) in den Hohlraum (5) zu drücken, über den Ausgang (30') der Hilfsöffnung (LX), um ein Blockieren der Position der Kanüle (1) im Hohlraum (5) zu ermöglichen,
der Ausgang (30') der Hilfsöffnung (LX) ist in einer vorher festgelegten Entfernung (d) vom Ausgang (20') der zusätzlichen Reperfusionsöffnung (LA) angeordnet, so dass, wenn der Anschlag (41) die Kanüle (1) an der Position im Hohlraum (5) blockiert, der Ausgang (20') der zusätzlichen Reperfusionsöffnung (LA) im Hohlraum (5) so ausgerichtet ist, dass die aufgefangene Flüssigkeit (F1) in der zweiten Richtung abgeleitet wird.

2. Injektionskanüle (1) nach dem vorangehenden Anspruch, **dadurch gekennzeichnet, dass** der vorher festgelegte Abstand (d) zwischen dem Ausgang (30') der Hilfsöffnung (LX) und dem Ausgang (20') der zusätzlichen Reperfusionsöffnung (LA) zwischen 0,1 und 500mm beträgt.

3. Injektionskanüle (1) nach irgendeinem der vorangehenden Ansprüche, **dadurch gekennzeichnet, dass** die Verschiebevorrichtung einen Drucktastenschalter (42) enthält, der in einer Längsnut (6) verschoben werden kann, die in der Kanüle (1) ausgespart ist, die Verschiebung des Drucktastenschalters (42) in der Längsnut (6) führt dann zur Verschiebung des Anschlags (41) entlang der Hilfsöffnung (LX) über ein Verbindungselement (43), das mit diesem Anschlag (41) verbunden ist.

4. Injektionskanüle (1) nach einem der vorangehenden Ansprüche, **dadurch gekennzeichnet, dass** der ausgefahrene Anschlag (41) Form und Maße aufweist, die so ausgelegt sind, dass das Zurückziehen der Kanüle (1) verhindert wird, wenn sie in den Hohlraum (5) eingeführt wird.

5. Injektionskanüle (1) nach einem der vorangehenden Ansprüche, **dadurch gekennzeichnet, dass** der Anschlag (41) aus einem Formgedächtnismaterial hergestellt ist.

6. Injektionskanüle (1) nach einem der vorangehenden Ansprüche, **dadurch gekennzeichnet, dass** der Anschlag (41) aus einem Draht aus Nickel- TitanLegierung hergestellt ist.

7. Injektionskanüle (1) nach einem der vorangehenden Ansprüche, **dadurch gekennzeichnet, dass** der ausgefahrene Anschlag (41) die Form einer Raute hat.

8. Injektionskanüle (1) nach einem der vorangehenden Ansprüche, **dadurch gekennzeichnet, dass** der Anschlag (41) eine thrombose- und/ oder wachstumshemmende Beschichtung aufweist.

9. Injektionskanüle (1) nach einem der vorangehenden Ansprüche, **dadurch gekennzeichnet, dass** der Anschlag (41) und / oder der Ausgang (30') der Hilfsöffnung (LX) eine strahlenundurchlässige oder schallundurchlässige Markierung aufweisen, die geeignet ist, eine Identifizierung dieses Anschlags (41) und/oder Ausgangs (30') dieser Hilfsöffnung (LX) durch Radiographie oder Echographie zu gewährleisten.

10. Injektionskanüle (1) nach irgendeinem der vorangehenden Ansprüche, **dadurch gekennzeichnet, dass** sie einen Ablasshahn (24) enthält, der mit der zusätzlichen Reperfusionsöffnung (LA) verbunden ist.

11. System zur Injektion einer Flüssigkeit in einen Hohlraum (5) des menschlichen oder tierischen Körpers, **dadurch gekennzeichnet, dass** es enthält:
- eine Einlasskanüle (2) zur Aufnahme einer vorher festgelegten Flüssigkeit; (FO);
- eine Pumpe (PMP) um das Pumpen der vorher festgelegten Flüssigkeit (FO), die aus der Einlasskanüle (2) stammt, zu gewährleisten;
- ein Oxygenator (OXY) zur Sicherstellung der Sauerstoffversorgung der vorher festgelegten Flüssigkeit (FO), die aus einem Ausgang der Pumpe (PMP) stammt,
- eine Injektionskanüle (1) nach irgendeinem der vorangehenden Ansprüche zum Injizieren der mit Sauerstoff angereicherten Flüssigkeit (F1) in den Hohlraum (5).

## Claims

1. Cannula (1) for injecting a fluid (F1) into a cavity (5) of the human or animal body, comprising:
- a main lumen (LP) defining a first space of the cannula (1) for the fluid (F1) to flow in a first direction; and
- an accessory reperfusion lumen (LA) defining a second space of the cannula (1) and comprising:
o an inlet (20) for collecting a portion of the fluid (F1) flowing in the first direction;
o an angled portion (21) for changing the direction of flow of the fluid (F1) collected by the accessory reperfusion lumen (LA); and
o an outlet (20') for discharging the collected fluid (F1) in a second direction into the cavity (5),
**characterized in that** the cannula (1) comprises a device for positioning the cannula (1) in the cavity (5), the positioning device comprising a stop (41) and movement means configured to move said stop (41) along an auxiliary lumen (LX), the auxiliary lumen (LX) defining a third space of the cannula (1) and comprising an outlet (30') opening into the cavity (5),
the movement means making it possible to deploy the stop (41) in the cavity (5), via the outlet (30') of the auxiliary lumen (LX), in order to ensure that the cannula (1) is locked in position in the cavity (5),
the outlet (30') of the auxiliary lumen (LX) being arranged at a predetermined distance (d) from the outlet (20') of the accessory reperfusion lumen (LA) such that when the stop (41) locks the cannula (1) in position in the cavity (5), the outlet (20') of the accessory reperfusion lumen (LA) is oriented in the cavity (5) such that the collected fluid (F1) is discharged in the second direction.

2. Injection cannula (1) according to the preceding claim, **characterized in that** the predetermined distance (d) between the outlet (30') of the auxiliary lumen (LX) and the outlet (20') of the accessory reperfusion lumen (LA) is between 0.1 and 500 mm.

3. Injection cannula (1) according to any one of the preceding claims, **characterized in that** the movement means comprise a push-button (42) capable of moving in a longitudinal groove (6) provided in the cannula (1), the movement of the push-button (42) in the longitudinal groove (6) causing the stop (41) to be moved along the auxiliary lumen (LX) via a connecting element (43) connected to said stop (41).

4. Injection cannula (1) according to one of the preceding claims, **characterized in that** the deployed stop (41) has a shape and dimensions designed to prevent the cannula (1) from being withdrawn when it is inserted inside the cavity (5).

5. Injection cannula (1) according to any one of the preceding claims, **characterized in that** the stop (41) is made from a shape memory material.

6. Injection cannula (1) according to any one of the preceding claims, **characterized in that** the stop (41) is made from a nickel-titanium alloy wire.

7. Injection cannula (1) according to any one of the preceding claims, **characterized in that** the deployed stop (41) is diamond-shaped.

8. Injection cannula (1) according to any one of the preceding claims, **characterized in that** the stop (41) has an anti-thrombogenic and/or anti-proliferative coating.

9. Injection cannula (1) according to any one of the preceding claims, **characterized in that** the stop (41) and/or the outlet (30') of the auxiliary lumen (LX) has/have a radio-opaque or echo-opaque marking designed to allow said stop (41) and/or the outlet (30') of said auxiliary lumen (LX) to be identified by radiography or echography.

10. Injection cannula (1) according to any one of the preceding claims, **characterized in that** it comprises a bleed valve (24) connected to the accessory reperfusion lumen (LA).

11. System for injecting a fluid (F1) into a cavity (5) of the human or animal body, **characterized in that** it comprises:
- an intake cannula (2) for receiving a predetermined fluid (F0);
- a pump (PMP) for pumping the predetermined fluid (F0) coming from the intake cannula (2);
- an oxygenator (OXY) for oxygenating the predetermined fluid (F0) coming from an outlet of the pump (PMP); and
- an injection cannula (1) according to any one of the preceding claims for injecting the oxygenated fluid (F1) into the cavity (5).
